# EUROPEAN PATENT SPECIFICATION

(11) **EP 0 505 846 B1**
(45) Date of publication and mention of the grant of the patent: **27.12.1995**
(21) Application number: 92104331.1
(22) Date of filing: 12.03.1992
(51) Int. Cl.: C07K 14/63, C07K 1/14

(54) **Process for converting fused protein into active type human motilin analogues and purifying the analogues**
Verfahren zum Umsetzen von Fusionsprotein in aktiven menschlichen motiunähnlichen Polypeptiden und Reinigung von diesen Analogen
Procédé pour la conversion d'une protéine fusionée en analogues actives de la motiline humaine et purification de ces analogues

(30) Priority: 27.03.1991 JP 63208/91
(43) Date of publication of application: 30.09.1992
(73) Proprietor: SANWA KAGAKU KENKYUSHO CO., LTD., Nagoya, Aichi-ken (JP)
(72) Inventor: Kurono, Masayasu, c/o SANWA KAGAKU KENKYUSHO CO., Nagoya, Aichi-ken (JP); Mitani, Takahiko, c/o SANWA KAGAKU KENKYUSHO CO., Nagoya, Aichi-ken (JP); Takahashi, Haruo, c/o SANWA KAGAKU KENKYUSHO CO., Nagoya, Aichi-ken (JP); Asano, Yukiyasu, c/o SANWA KAGAKU KENKYUSHO CO., Nagoya, Aichi-ken (JP); Nakoshi, Masanao, c/o SANWA KAGAKU KENKYUSHO CO., Nagoya, Aichi-ken (JP); Tanaka, Mika, c/o SANWA KAGAKU KENKYUSHO CO., Nagoya, Aichi-ken (JP); Kobara, Yukari, c/o SANWA KAGAKU KENKYUSHO CO., Nagoya, Aichi-ken (JP); Sawai, Kiichi, c/o SANWA KAGAKU KENKYUSHO CO., Nagoya, Aichi-ken (JP)
(74) Representative: Wächtershäuser, Günter, Prof. Dr.

(56) References cited:
- EP-A- 0 355 720
- EP-A- 0 378 078
- BIOTECHNOLOGY LETTERS vol. 10, no. 11, November 1988, KEW, SURREY, GB pages 763 - 768; ETSUKO, MIYASHITA ET AL.: 'High level production of a peptide hormone analogue (Leu13)Motilin in E. coli'

## Description

The present invention relates to a process for converting a fused protein of motilin analogues to an active form and a method of purification of the analogues, and more particularly converting the fused protein containing a tandemly repeated human motilin analoques expressed in transformed microorganisms to a biologically active form of human motilin analogues and purifying the motilin analogues in such an extent that the analogue can be used as an effective ingredient for preparing a medicine.

Motilin is one of peptide hormones, firstly isolated from mucosa of porcine small intestine, and determined its amino acid sequence by Brown J. C. et al ["Gastroenterology", Vol. 62, pages 401 - 404 (1972) and " Can. J. Biochem.", Vol. 52, pages 7 - 10 (1974)]. The porcine motilin consists of 22 amino acid residues and has molecular weight of about 2700.

While, some of the present inventors have succeeded in an isolation of cloned cDNA encoding human motilin precursor, the nucleotide sequence therein including a part coding the amino acid sequence same with that for the porcine motilin, so that it has been made apparent that the human motilin has the amino acid sequence same with that for the porcine motilin [Jap. Pat. No. Sho 63 - 276489(A) which corresponds to United States Pat. Appln. Ser. No. 07/190849 and European Pat. Publn. No. 0289995(A1)].

As physiological activities of the motilin, a hypermotility action of digestive tract and contraction of gastroduodinal and colonic smooth muscle therein have been well known. As the hypermotility action, it has been reported that the rate of gastric emptying is shortened ["Gastroenterology", Vol. 80, pages 456 - 460 (1981)] and as the contraction of smooth muscle in the digestive tract, it has been known that the motilin shows a strong contraction to a rabbit and human gastrointestinal tract, independent from a neuro system. Moreover, no report has been issued on any specific side-effect. Therefore, it has been considered that the motilin is useful for curing gastroenteropathys at the period of post-operation and for diagnosis thereof. In connection with this, please note that prostagrandin has widely been employed for curing the gastroenteropathys, although the drug shows a relatively strong side-effect.

It has also been reported that a chemically synthesized motilin analogue --methionine residue at 13-position of native motilin being substituted with leucine or norleucine residue--shows biological activities similar to pure native porcine motilin ["Scand. J. Gastroenterology", Vol. 11, pages 119 - 203 (1976) and others]. Therefore, it has been considered that methionine residue at 13-position has almost no influence on useful activities of the motilin.

Further, the present inventors have made it apparent that motilin analogues with homoserine or homoserine-lactone residue at C-terminal show biological activities in same level with or higher than the native motilin [Jap. Pat. Appln. No. Sho 64 (1989) - 286 corresponding to a part of United States Pat. Appln. Ser. No. 07/459236 and European Pat. Publn. No. 0378078(A1)]. In these literatures, there is given a process for preparing the motilin analogues with reasonable cost by utilizing recombinant DNA techniques.

The motilin according to widely accepted technical art has been obtained through an extraction from porcine organ tissue and thus it was quite difficult to obtain the same in large amount. Since the motilin is polypeptide consisting of 22 amino acid residues, a large scale production is difficult and a cost thereof becomes higher, even if a chemical synthesis shall be applied therefor. Namely, the motilin has not actually been employed for clinical use, due to its poor productivity, in spite of that an effectiveness thereof as the drug for curing gastroenteropathys and others have been expected.

Therefore, various studies have been made for preparing polypeptides having motilin-like biological activities, with reasonable cost, by utilizing the so-called "Biotechnology" [Jap. Pat. Nos. Sho 63 (1988)- 71195(A) and 63 (1988) - 208006(A) which corresponds to United States Pat. Appln. Ser. No. 07/390149 and European Pat. Publn. No. 0355720(A1)].

All of said processes utilizing the biotechnology comprise a step for cleaving a polypeptide chain of fused protein with use of cyanogen bromide, and a step for removing an excess peptide chain containing homoserine or homoserine-lactone residue at C-terminal, with use of an expensive enzyme(s). However, the enzymatic treatment reduces yield of a desired motilin analogue to increase a cost thereof, which makes difficult a large scale production of the motilin analogue. In connection with this, the present inventors have found that as aforesaid, the polypeptides per se with homoserine or homoserine-lactone residue at C-terminal, which can be obtained through the cleaving operation by cyanogen bromide, show biological activities in same level with or higher than native pure motilin [said Jap. Pat. Appln. No. Sho 64 (1989) - 286 corresponding to a part of said United States Pat. Appln. Ser. No. 07/459236 and European Pat. Publn. No. 0378078(A1)]. Further, the present inventors have succeeded preparation of an expression plasmid with an insert encoding the polypeptide with methionine residue at C-terminal and of a host of microorganism (Escherichia coli) transformed by the plasmid to develop a process for preparing the motilin analogues in more easy and with reasonable cost [Jap. Pat. Appln. No. Hei 1 (1989) - 216034 corresponding to a part of said United States Pat. Appln. Ser. No. 07/459236 and European Pat. Publn. No. 0378078(A1)].

A principal object of the invention is to provide a process for converting a fused protein comprising a polypeptide expressed in transformed microorganisms into individual biologically active type human motilin analogues and purifying the active type human motilin analogues in such an extent that it can be used as an ingredient for preparing a medicine.

An additional, but important object of the present invention is to provide a process for preparing human motilin analogues with good efficiency and reasonable cost to make possible an industrial scale production thereof.

The present inventors have already established a system for expressing as a fused protein in transformed microorganisms a polypeptide which encodes plural human motilin analogues in tandem arrangement. According to the former method developed by the inventors, the fused protein is purified, if necessary after dissolved with a denaturating agent, and then treated with cyanogen bromide in a acid solution such as 70% formic acid solution to cleave the fused protein into individual active type polypeptide fragments, each encoding the motilin analogue gene. However, resulting reaction solution can not be subjected to a conventional protein separating operation, such as various chromatographies. In other words, the formic acid should be removed prior to the protein separating operation for obtaining a desired polypeptide encoding human motilin analogue. As a method of removing formic acid, it may be thought to neutralize the reaction solution with an alkali such as sodium hydroxide, remove insoluble materials therein, and gel-filtlate the solution. However, this method is not convenient in that the gelfiltration of solution including cyanogen bromide provides a large amount of toxic waste solution and an equipment for separating the neutralizing solution in large amount is required for industrial large scale production. Therefore, the reaction solution has been lyophilized to remove the formic acid.

When the fused protein is cleaved into the polypeptide fragments with use of cyanogen bromide, there are many cases of that undesired analogues are formed in addition to the desired polypeptide fragments. It is difficult to separate the undesired analogues from the desired polypeptide fragments by utilizing a conventional ion-exchange chromatography or the like low-pressure column chromatography and thus a high performance liquid chromatography (HPLC) is required. In this case, an elution method utilizing a gradient in concentration of an organic solvent gives a limitation in an amount of the separable polypeptide. While, an isochoric method using an organic solvent in constant concentration allows a separation in relatively large amount but gives a limitation in an amount of the separable polypeptide within a unit period of time, since an elution solvent is required in large amount and necessary elution period of time becomes longer.

Therefore, the inventors have further studied and investigated as to a simple method for isolating the desired polypeptide from the fused protein expressed in transformed microorganisms and purifying the same in such an extent that the polypeptide encoding the motilin analogue can be used as an effective ingredient for medicines to find that a crude product of the desired polypeptide can be obtained by treating the microorganisms per se which contains the fused protein with cyanogen bromide, neutralizing reaction solution with an alkali such as sodium hydroxide to make insoluble and remove various undesired materials derived from the microorganisms, subjecting resulting supernatant to a hydrophobic chromatography for desalting. These operations can be carried out in a short period of time and without requiring any special equipment, even if the production scale shall be larged into industrial one. They have further found that the crude polypeptide can easily be adsorbed to a resinous carrier for ion-exchange chromatography by electrodializing the crude solution with use of an ion-exchange membrane, and that the desired motilin analogue can be refined to a desired purity by carrying out an ion-exchange chromatography and then a reversed phase chromatography which utilizes a two-staged isochoric elution method using two solvents different in concentration thereof, so that the invention has been established.

The invention provides a process for preparing in industrial scale a motilin analogue shown by following Formula I. 
Phe-Val- A -Ile-Phe-Thr-Tyr- B -Glu-Leu- C -Arg- X - Gln-
Glu-Lys-Glu-Arg- D -Lys-Gly- E - Y
wherein A is a residue of amino acids selected from the group consisting of Pro, Gly, Asn and Ser; B is a residue of amino acids selected from the group consisting of Gly, Pro, Asn and Ser; C is a residue of amino acids selected from the group consisting of Gln, Glu and Asp; D is a residue of amino acids selected from the group consisting of Asn, Glu and Asp; E is a residue of amino acids selected from the group consisting of Gln, Lys and Arg; X is a residue of amino acids other than Met; and Y is a residue of homoserine (inclusive of homoserine-lactone and designated by a symbol of "Hse") or an optional polypeptide consisting of amino acid residues not exceeding 10 and Hse at C-terminal.

As aforesaid, the inventors have established a system for expressing as the fused protein in transformed microorganisms a polypeptide which encodes an amino acid sequence similar to Formula I in plurality and there is methionine residue between the neighboring amino acid sequences [see said Jap. Pat. Appln. No. Sho 64 (1989) - 286 corresponding to United States Pat. Appln. Ser. No. 07/459236 and European Pat. Publn. No. 0378078(A1)]. Each of the polypeptides can be obtained by cleaving the fused protein with cyanogen bromide. In connection with this, please note that the cyanogen bromide treatment causes cleavage the fused protein at Met residue position therein to convert the Met residue to Hse residue.

The process according to the invention and for converting the fused protein expressed in the transformed microorganisms and comprising a polypeptide which encodes plural human motilin analogues in tandem arrangement into individual active type motilin analogues and highly purifying the active type motilin analogues, comprises steps of sterilizing and defating the microorganisms expressed the fused protein therein; filtering the same to obtain sterilized microorganisms; crushing the same to obtain an inclusion body; dissolving the inclusion body in an aqueous solution of formic acid, adding cyanogen bromide to cleave the fused protein into individual polypeptide fragments, each of which encodes the motilin analogue; neutralizing resulting reaction mixture with an alkali; centrifuging the reaction mixture to remove solids; subjecting a supernatant to a hydrophobic chromatography; eluting with an acidic aqueous solution or water to obtain a fraction containing the polypeptide fragments; desalting the fraction; subjecting resulting solution to an ion-exchange chromatography to obtain a fraction containing the polypeptide fragments; subjecting the fraction to a high performance liquid chromatography with use of two acidic aqueous solutions, each containing acetonitrile but different in its concentration; concentrating and lyophilizing a fraction; and then desalting the fraction.

In the process of invention, the microorganisms containing the expressed fused protein can be crushed by radiating ultrasonic wave thereto or with use use of a high pressure homogenizer, and centrifuge the same to obtain a solid. The solid is treated with the solution containing formic acid. It is preferable to use the formic acid with concentration of 50 - 80% by weight. It is unnecessary to separate at this stage a liquid phase containing the desired polypeptide and undesired materials remaining as solids, since the undesired materials can be removed through the centrifugal treatment to be carried out after the subsequent neutralizing operation. As the alkali, sodium hydroxide, potassium hydroxide or the like can be used. It is preferable to set a pH of the neutralized mixture to 3 - 3.5. If the pH is higher than 3.5, an amount of insoluble materials becomes larger to cause lowering yield of the desired polypeptide, due to high concentration of salts therein. While, if the pH is lower than 3, the mixture may cause a corrosion of a vessel such as a stainless-steel container and there is a possibility of insufficient precipitation of undesired materials. However, if the pH of the neutralized mixture is lower than 10, the undesired materials derived from microorganisms can be precipitated to remain the desired polypeptide in liquid phase, by setting concentration of the salt in a low level, but in this case, an amount of solution containing toxic cyanogen bromide becomes larger and an addition of a salt such as ammonium sulfate will be required to make possible adsorption of the desired polypeptide on a carrier in subsequent step of hydrophobic chromatography.

The solution neutralized and removed solids therefrom through a centrifugal separation or the like is applied to the column of hydrophobic chromatography, which carrier, in general, is packed in a column. As the carrier, it is preferable to select those rich in alkyl group such as butyl, octyl or the like, or phenyl functional group and with a support of polyvinyl or the like synthetic polymer, or dextran, agarose, cellulose or the like polysaccharide. As marketed carrier therefor, followings may be listed; Butyl Toyo Pearl, Octyl Sepharose CL-4B, Phenyl Sepharose, Butyl Sepharose 4B, Agarose-hexane, Agarose-octylamine, Sepabeads FP-PH, Sepabeads FP-OT and Sepabeads FP-Bu. Among them, Butyl Toyo Pearl is most preferable.

For causing the elution of desired polypeptide in the hydrophobic chromatography, it is sufficient only by lowering a concentration of salt therein. Therefore, refined water can be used as eluate therefor, and it is preferable to set pH of the solution to a value away from isoelectric point of the polypeptide to be purified. If necessary and there is no bad influence on subsequent separating operation, a hydrophilic organic solvent such as acetonitrile may be added. An eluate has a relatively high salt concentration and thus desalting thereof shall be carried out with a gel-filtration, electrodialysis or the like method.

When the electrodialysis is employed, it is preferable to use a semipermeable membrane having a fractional molecular weight of 100 - 500, and to add a salt in outer dialyzing solution to suppress a decrease of the yield of desired polypeptide. It is not preferable to use a semipermeable membrane having a fractional molecular weight higher than 1000, since the membrane allows a permeation of the desired polypeptide to cause lowering of the yield.

With the aforesaid operations, a crude product of the desired polypeptide (motilin analogue) can be obtained. The crude product can be refined to obtain a highly pure motilin analogue by directly subjecting the same to a high performance liquid chromatography (HPLC) or the like separation method with a high theoretical plate number, but such separation method is not preferable for efficient production of the desired polypeptide. Therefore, according to the invention, at least one ion-exchange chromatography is carried out prior to the HPLC. Namely, the desalted eluate was adjusted in pH to 2 to 5 by adding an acid such as acetic acid or an acidic buffer and then subjected to a cation-exchange chromatography.

Various resin marketed for purifying proteins can be used for the cation-exchange chromatography. it is preferable to select the carrier rich in carboxymethyl or sulfonic exchanging radical and with a support of dextran, agarose, cellulose or the like polysaccharide, polyacrylate, hydrophilic polyvinyl or the like synthetic high molecular weight substance. Most preferable cation-exchange resin is S-Sepharose FF with sulfonic functional group. When the pH adjusted eluate was charged into a column packed with the sulfonic cation-exchange resin, the desired polypeptide will be eluted at a value of pH higher than 6. Therefore, an aqueous salt solution such as saline in concentration gradient of 0.01 - 0.5M, increasing its concentration or pH in stepwise to cause an elution of the desired polypeptide.

An analysis of resulting eluate utilizing a reversed phase HPLC with octadecyl (C-18) type ODS carrier (pH : about 2, gradient elution of acetonitrile) shows that firstly eluting fractions are main fractions of the desired polypeptide and contain undesired other polypeptides in a small amount to remarkable refine the same prior to the ion-exchange chromatography.

The eluate may be directly subjected to HPLC, but it is preferable to carry out, prior to the HPLC, an operation for opening lactone ring of homoserine residue at C-terminal of the desired polypeptide to convert the same into homoserine residue. For this purpose, the eluate is made into a basic solution. It is preferable to set pH of the eluate to 9 - 11 and more preferably less than 10 to avoid a possible decomposition of the desired polypeptide.

The pH controlled eluate is subjected to a reversed phase HPLC. Namely, the eluate is charged into a column packed with ODS carrier for HPLC for adsorption of the desired polypeptide. An eluation of the polypeptide is carried out in 2 staged method. In the first stage, an aqueous solution of acetonitrile in lower concentration (18 - 20% by weight) is used to remove firstly eluting fractions of undesired substance(s) and obtain the desired fraction. In the second stage, an aqueous solution of acetonitrile in higher concentration (higher than the first solution but lower than 30 % and preferable range is 20 - 24%) is used to cause an elution of remaining desired polypeptide. If the concentration of the second solution is made higher, an amount thereof required for the elution decreases, but an amount of substance(s) other than the desired polypeptide increases in resulting eluate. It is preferable to set pH of the first and second solutions to 2.0 - 9.0, but 2 - about 6 is preferable, when silica type ODS carrier is employed. This 2 staged operation is preferable than a method using an eluent with constant concentration in that required period of time can be shorten and required amount of the eluent decreases.

The resulting acidic eluate is neutralized at once to avoid a possible decomposition of the desired polypeptide. The neutralized eluate is concentrated and desalted with methods known per se to afford the desired polypeptide with high purity (Purity : 99% or more).

The polypeptide showing motilin-like biological activities is in the form of solution which can be lyophilized for storing and other purposes.

The polypeptides prepared by the process according to the invention can be employed as an effective ingredient for various medicines for curing gastroenteropathys, improving abnormal secretion of tear and others. There is no specific limitation, when the polypeptide will be made into the medicine. Therefore, the medicine may be of a solid form such as tablet, pill, capsule, powder, granule and suppository, or a liquid form of solution, suspension or emulsion, together with a conventional additive(s) and/or a carrier(s). If necessary, a stabilizer such as albumin or the like may be composed.

The invention will now be further explained with reference to an Example.

In the followings, the explanation will be given on a motilin analogue, in which methionine residue at 13-position of native motilin is modified into leucine residue and having Hse residue at 23 position {[L-Leu¹³]-motilin-Hse}, but please note that other and various motilin analogues having an amino acid residue other than methionine residue at 13-position, can be prepared in a similar manner.

### Example

It was taken about 350g of acetone powders of recombinant microorganisms (Escherichia coli) containing a fused protein of tandemly arranged motilin analogues. The acetone powder was suspended in 80 litres of saline and treated by a high pressure homogenizer at pressure condition higher than 400kg/cm² to crush or break the body of microorganisms. Resulting solution was continuously centrifuged by a Drabal type centrifugal machine to concentrate the solution to 10 litres. To the concentrated solution, sucrose and refined water were added to prepare 0.5M sucrose solution which was centrifuged to recover insoluble precipitate, as pellets. The pellets contains therein the fused protein.

The pellets were suspended in refined water to make its volume of 8.6 litres. In the suspension, formic acid (20 litres) was added to dissolve the solid therein, and then cyanogen bromide (75g) was added to cause a reaction one overnight at about 30°C. After addition of refined water (3 litres) to the reaction solution and while cooling the same with chilled water, 5N solution (40 litres) of sodium hydroxide was gradually added thereto to left for stand for about 1 hour. Then, a precipitate was removed by a centrifugal treatment using a filter with pore size of 0.3 »m to obtain a solution.

After bufferized with 50% saturated solution of ammonium sulfate a column (9cm x 80cm) packed with a carrier of Butyl Toyo Pearl 650M (Trademark) for hydrophobic chromatography, the solution was charged to cause an adsorption of [L-Leu¹³]-motilin-Hse on the carrier, and then charged 0.5% aqueous solution of acetic acid to cause an eluation of the polypeptide for obtaining fractions, main peak of which is detected by measuring an absorbance at 280nm.

The fractions were combined and desalted by an electrodializer (marketed by Asahi Chemical Industry Co. Ltd. of Japan as "Microasylizer G3") with use of a styrenic membrane having a fractional molecular weight of 300 and effective membrane area of 400cm² as well as an aqueous solution containing ammonium sulfate by about 1% by weight, as an outer solution for the dialysis, until resulting dialized solution shows a current value of 0.1A at voltage of 7.5V. The desalted solution was charged into a column (14cm x 20cm) packed with a residue for cation exchange chromatography of S-Sepharose FF, which column was previously bufferized with 0.5% aqueous acetic acid solution. The column was washed with 100mM sodium acetic acid buffer (pH 5.0, containing 50mM NaCl) and then eluted with the same kind buffer but containing 350mM NaCl to obtain fractions containing the desired [L-Leu¹³]-motilin-Hse, main peak of which can be detected by measuring an absorbance at 280nm.

To resulting eluate, 5N sodium hydroxide was gradually added to adjust pH to about 10 and treated for 1 hour with use of a water bath kept at about 37°C.

From the neutralized solution, 100ml thereof were taken. The solution was charged into a column packed with ODS silica carrier (5cm x 25cm, pore size 120Å, particle size 10 »m) which was previously bufferized with 19% aqueous acetonitrile solution containing 0.012N HCl, and then the bufferizing solution was charged into the column at flow ratio of 60ml/min to cause elution of materials adsorbed on the carrier. In this case, an elution of the objective [L-Leu¹³]-motilin-Hse was checked by continuously measuring an absorbance with use of a UV detector (beam length : 3mm) and at 220nm. Namely, when an absorbance indicated by the detector reached 0.1, the elution solvent was changed to 22% aqueous acetonitrile solution containing 0.012N HCl to obtain a fraction showing absorbance of 0.5 or more, which contains the [L-Leu¹³]-motilin-Hse, and then a phosphate buffer (pH 7.5) was added, at once, to the fraction for neutralization thereof.

The neutralized eluates obtained were combined, diluted with refined water by twice in volume, and charged into a column of Prepack Cartridge Delta Pack ("Trademark", marketed by Waters Co., 4.7cm x 30cm, particle size : 15 »m, functional group : C18, pore size : 100Å) bufferized previously with 0.003N HCl. The column was washed with the buffer solution, and an elution was carried out with use of 30% aqueous acetonitrile solution containing 0.002N HCl, to obtain a fraction in main peak part containing the [L-Leu¹³]-motilin-Hse, which fraction was neutralized at one by adding thereto a phosphate buffer (pH 7.5), so that final concentration of the buffer becomes 10mM.

All of neutralized fractions were combined, concentrated with use of an evaporator, and lyophilized to remove almost all of the solvents. Resulting polypeptide powder was dissolved in refined water to obtain about 2% aqueous solution. The solution was desalted by an electrodialysis which uses a membrane with a fractional molecular weight of 300 and effective area of 400cm² as well as a solution containing about 1% ammonium sulfate, as outer solution, until dialized solution shows a current value less than 0.01A at voltage of 7.5V.

Resulting dialyzed solution contained 20g or more of the desired [L-Leu¹³]-motilin-Hse with purity of 99% or more. The quantitative analysis according to the Limulus Test showed that endotoxin of the motilin analogue is a level lower than identification limit.

## Claims

1. A process for converting a fused protein containing a tandemly repeated human motilin analogue expressed in transformed microorganisms to biological active form of motilin analogues and purifying the active type human motilin analogues, which process comprises steps of sterilizing the transformed microorganisms expressed the fused protein therein; filtering the same to obtain sterilized microorganisms; crushing the same to obtain an inclusion body; dissolving the inclusion body in an aqueous solution of formic acid, adding cyanogen bromide to cleave the fused protein into individual polypeptide fragments, each of which encodes the motilin analogue; neutralizing resulting reaction mixture with an alkali; centrifuging the reaction mixture to remove solids; subjecting a supernatant to a hydrophobic chromatography; eluting with an acidic aqueous solution or water to obtain a fraction containing the polypeptide fragments; desalting the fraction; subjecting resulting solution to an ion-exchange chromatography to obtain a fraction containing the polypeptide fragments; subjecting the fraction to a high performance liquid chromatography with use of two acidic aqueous solutions, each containing acetonitrile but different in its concentration; concentrating and lyophilizing a fraction; and then desalting the fraction.

2. A process as claimed in Claim 1, wherein pH of said neutralized solution is not higher than 10, and more preferably in the range of 3 - 3.5.

3. A process as claimed in Claim 1, wherein said hydrophobic chromatography is carried out with use of a column containing an alkyl or phenyl functional group.

4. A process as claimed in Claim 1, wherein each of said desalting operations is carried out with use of an electrodializer with an ion-exchange membrane.

5. A process as claimed in Claim 1, wherein said ion-exchange chromatography is cation exchange one and is carried out with use of a column containing a carboxymethyl or sulfonic functional group.

6. A process as claimed in Claim 1, wherein said high performance liquid chromatography is carried out with use of a column containing a reversed phase functional group.

## Patentansprüche

1. Vefahren zum Umwandeln eines Fusionsproteins, enthaltend ein tandemartig wiederholtes, menschliches Motilin-Analoges, das in transformierten Mikroorganismen exprimiert ist, in die biologisch aktive Form von Motilin-Analogen und Reinigen der aktiven, menschlichen Motilin-Analogen, umfassend die Stufen des Sterilisierens der transformierten Mikroorganismen mit dem exprimierten Fusionsprotein darin; Filtrieren derselben, um sterilisierte Mikroorganismen zu erhalten; Zerkleinern derselben, um einen Einschlußkörper zu erhalten; Auflösen des Einschlußkörpers in einer wässerigen Lösung von Ameisensäure; Hinzugeben von Bromcyan, um das Fusionsprotein in einzelne Polypeptid-Fragmente zuspalten, deren jedes für das Motilin-Analoge codiert; Neutralisieren der resultierenden Reaktionsmischung mit einem Alkali; Zentrifugieren der Reaktionsmischung zur Entfernung von Feststoffen; Ausführen einer hydrophoben Chromatographie am Überstehenden; Eluieren mit einer sauren, wässerigen Lösung oder Wasser, um eine die Polypeptid-Fragemente enthaltende Fraktion zu erhalten; Entsalzen der Fraktion; Ausführen einer Ionenaustausch-Chromatographie an der resultierenden Lösung, um eine die Polypeptid-Fragmente enthaltende Fraktion zu erhalten; Ausführen einer Hochleistungs-Flüssigkeits-Chromatographie an der Fraktion unter Einsatz von zwei sauren, wässerigen Lösungen, die jeweils Acetonitril, aber in unterschiedlicher Konzentration, enthalten; Konzentrieren und Lyophilisieren einer Fraktion und dann Entsalzen der Fraktion.

2. Verfahren nach Anspruch 1, worin der pH der neutralisierten Lösung nicht höher als 10 ist und bevorzugter im Bereich von 3 bis 3,5 liegt.

3. Verfahren nach Anspruch 1, worin die hydrophobe Chromatographie unter Einsatz einer funktionelle Alkyl- oder Phenylgruppen enthaltenden Säule ausgeführt wird.

4. Verfahren nach Anspruch 1, worin jede der Entsalzungsstufen unter Einsatz eines Elektrodialysators mit einer Ionenaustauschmembran ausgeführt wird.

5. Verfahren nach Anspruch 1, worin die Ionenaustausch-Chromatographie eine Kationenaustausch-Chromatographie ist und diese unter Einsatz einer funktionelle Carboxymethyl- oder Sulfonsäuregruppen enthaltenden Säule ausgeführt wird.

6. Verfahren nach Anspruch 1, worin die Hochleistungs-Flüssigkeits-Chromatographie unter Einsatz einer Säule ausgeführt wird, die funktionelle Umkehrphasen-Gruppen enthält.

## Revendications

1. Procédé pour convertir une protéine de fusion, contenant un analogue de la motiline humaine à séquences répétées en tandem, exprimée dans des microorganismes transformés, en une forme biologiquement active d'analogues de la motiline, et pour purifier les analogues de la motiline humaine du type actifs, lequel procédé comprend les étapes consistant à stériliser les microorganismes transformés qui expriment la protéine de fusion ; à filtrer ceux-ci pour obtenir des microorganismes stérilisés ; à broyer ceux-ci pour obtenir un corps d'inclusion ; à dissoudre le corps d'inclusion dans une solution aqueuse d'acide formique, à ajouter du bromure de cyanogène pour cliver la protéine de fusion en fragments polypeptidiques individuels, chacun d'entre eux codant pour l'analogue de la motiline ; à neutraliser avec un alcali le mélange réactionnel obtenu ; à centrifuger le mélange réactionnel pour éliminer les matières solides ; à soumettre le surnageant à une chromatographie hydrophobe ; à éluer avec une solution aqueuse acide ou avec de l'eau pour obtenir une fraction contenant les fragments polypeptidiques ; à dessaler la fraction ; à soumettre la solution obtenue à une chromatographie par échange d'ions pour obtenir une fraction contenant les fragments polypeptidiques ; à soumettre la fraction à une chromatographie en phase liquide haute performance à l'aide de deux solutions aqueuses acides, chacune contenant de l'acétonitrile mais à des concentrations différentes ; à concentrer et à lyophiliser la fraction ; puis à dessaler la fraction.

2. Procédé selon la revendication 1, dans lequel le pH de ladite solution neutralisée n'est pas supérieur à 10, et est de préférence de 3 à 3,5.

3. Procédé selon la revendication 1, dans lequel ladite chromatographie hydrophobe est effectuée à l'aide d'une colonne contenant un groupe fonctionnel alkyle ou phényle.

4. Procédé selon la revendication 1, dans lequel chacune desdites opérations de dessalage est effectuée à l'aide d'un appareil d'électrodialyse avec une membrane d'échange d'ions.

5. Procédé selon la revendication 1, dans lequel ladite chromatographie par échange d'ions est une chromatographie par échange de cations et est effectuée à l'aide d'une colonne contenant un groupe fonctionnel carboxyméthyle ou sulfonique.

6. Procédé selon la revendication 1, dans lequel ladite chromatographie en phase liquide haute performance est effectuée à l'aide d'une colonne contenant un groupe fonctionnel à inversion de phases.
